# EUROPEAN PATENT APPLICATION

(11) **EP 4 491 189 A1**
(43) Date of publication of application: **15.01.2025**
(21) Application number: 23767006.2
(22) Date of filing: 06.01.2023
(51) Int. Cl.: A61K 38/08, A61P 1/02, A23L 33/17

(54) **COMPOSITION FOR PREVENTING OR TREATING DENTAL CARIES**

(30) Priority: 08.03.2022 KR 20220029535
(71) Applicant: Hysensbio Co., Ltd, Gwacheon-si Gyeonggi-do 13814 (KR)
(72) Inventor: PARK, Joo Hwang, Incheon 21986 (KR); LEE, Dong Seol, Seoul 03088 (KR); GUG, Hye Ri, Seoul 02811 (KR)
(74) Representative: Mewburn Ellis LLP
(86) International application number: PCT/KR2023/000315
(87) International publication number: WO 2023/171900

(57) **Abstract**

The present invention relates to a pharmaceutical composition for preventing or treating dental caries and the use thereof. The pharmaceutical composition, according to the present invention, includes a peptide comprising an amino acid sequence of General Formula 1 as an active ingredient:

K-Y-R1-R2-R3-R4-R5-R6-R7-R8 (General Formula 1).

In General Formula 1,
R1 is arginine (R), lysine (K), or glutamine (Q),
R2 is arginine (R) or glutamine (Q),
R3, R4, and R5 are each arginine (R) or lysine (K),
R6 is asparagine (N) or serine (S), and
R7 and R8 are lysine (K) or tyrosine (Y).

## Description

### [Technical Field]

The present invention relates to a pharmaceutical composition, particularly a pharmaceutical composition for preventing or treating dental caries without involving the removal of infected dentin.

### [Background Art]

Dental caries is one of the most common chronic diseases, and it has been reported that 3.5 billion people worldwide are affected (Dye 2017; Kassebaum et al. 2015; World Health Organization 2017). According to the 2013 data from the Ministry of Health and Welfare of the Republic of Korea, the rate of permanent tooth caries in adult males was approximately 87%, and in females, approximately 90%, indicating a high prevalence rate. Due to the overall development of medical technology, the average life expectancy of people has been extended, and the importance of oral health management to improve the quality of life is increasing, and above all, the importance of preserving natural teeth is being emphasized. Early dental caries is asymptomatic, but as it progresses, it causes various types of pain and eventually tooth loss, and ultimately, the edentulous state causes eating disorders, which is a factor that significantly lowers the overall quality of life (Bortoluzzi et al. 2012).

In general, when the dentin is damaged due to non-infectious causes, a simple process of restoring the lost dentin is required, but in the case of dental caries, the treatment method is different from that for dentin diseases caused by other non-infectious causes in the sense that the impact of bacteria, the cause of the affected dentin and infections, should be minimized. Dental caries, unlike other dentin-pulp diseases, begins with infection of the dentin by the causative bacteria of dental caries. Since dental caries is an infectious disease caused by bacteria, it is important to minimize the impact of bacteria by removing the affected tooth, the conventional treatment for dental caries generally involves removing the infected dentin to physically remove the source of bacterial infection and restoring the removed dentin region.

The general treatment methods for dental caries that are currently carried out have problems such as delayed treatment due to fear of dental treatment and problems arising from using artificial dental materials.

Teeth are composed of hard tissues such as enamel, dentin, cementum, and pulp, which is an internal soft tissue. Nerves and blood vessels are developed in the pulp, and odontoblasts that form dentin are located at the boundary between the pulp and dentin. Considering the erosion of the components that make up the teeth, dental caries may be divided into five stages according to the degree of progression. Specifically, stage 1 of dental caries is a state in which minor damage limited to the enamel has almost no symptoms, stage 2 involves damage near the boundary between enamel and dentin and is accompanied by symptoms of sensitive teeth, stage 3 is a stage in which the dentin is affected and damaged and pain can be felt due to external stimuli, stage 4 is a stage in which the pulp is affected and inflammation is caused even in the absence of external stimuli, and stage 5 is a stage in which necrosis of the pulp and severe pain are present. Generally, dental caries is often recognized only in stages 2 and 3, which are the early stages. Stages 2 and 3 may be an appropriate time to treat dental caries by minimizing the impact of the causative bacteria of dental caries.

Although dental treatment may be quickly received through subjective symptoms, it is known that the appropriate treatment stage is often missed, and this is due to the traditional treatment method for dental caries. Current treatment of dental caries is a process of mechanically removing weakened dentin and filling the region with new material, so most dental patients tend to delay treatment due to anxiety, worry, fear, and nervousness about dental treatment. Therefore, in some cases, the appropriate treatment time is missed, and a significant portion of the dentin should be removed during the treatment process. Anxiety or worry about dental caries treatment is found in various age groups, from young children to the elderly. In particular, infants and adolescents (0 to 14 years old) who are not yet psychologically and physically mature are known to have a great fear of dental treatment. In 1984, Berggren and Meynert investigated and reported the causes of avoidance of dental treatment in adults aged 20 to 40, and the results showed that approximately 85% of the subjects had developed a fear of dental treatment due to traumatic dental treatment experiences in their childhood, and the pain experienced during cavity formation, anesthesia, or tooth extraction was pointed out as the main cause of the fear of dental treatment. The fear of dental treatment formed in childhood may continue into adulthood, so it may be a factor in missing the appropriate treatment time.

The dental materials used in the conventional treatment of dental caries are various restorative and prosthetic restorative materials composed of various ingredients such as amalgam, glass ionomer cement, composite resin, gold, and porcelain to restore the structure and function of teeth from dental damage. The restorative materials used in dental treatment so far have a high possibility of causing microleakage in the long term, and so the chemical stimulation and microleakage of dental materials for teeth preservation and prosthetic restoration may cause or worsen lesions in the pulp. When the inflammatory reaction progresses as a result, it leads to pulp necrosis, resulting in loss of dentin due to root canal treatment to remove the pulp and additional tooth removal. Root caries, which often occurs in the elderly, makes it difficult to prevent moisture, secure a field of vision, and access during restoration, and since the margin of the cavity is located in the cementum and dentin, it is reported that in root caries, unlike the caries in the enamel, the resistance to acid erosion is weak, and recurrence of caries commonly occurs due to incomplete margins. In addition, in the case of regions where it is difficult to maintain the restored shape and the restoration may easily fall off, since the dentin thickness from the root to the pulp is not thick, the frequency of pain occurrence after treatment is high, and when the dental caries is removed, the pulp is easily exposed, so it often leads to root canal treatment, making it difficult to preserve the natural tooth.

In the early stage of dental caries, the patient may feel a little pain or no pain at all. Since dental caries has not progressed to the pulp where the nerves and blood vessels are located inside the tooth, only the infected part is removed and filled with a dental material. However, when dental caries have invaded the pulp, root canal treatment, commonly referred to as nerve treatment, is necessary. Root canal treatment is when all the nerves and blood vessels inside the pulp are removed, and the regions are filled with dental material. After root canal treatment is finished, the removed part of the tooth is filled with dental material. Teeth that have undergone root canal treatment are likely to break with the filling treatment alone, so after the filling treatment, they are covered with a gold crown made by casting a metal. When dental caries progresses to the pulp and continues for a long time, an abscess will be formed in the bone at the tip of the root, and the pus will flow through the bone and swell the gums. Root canal treatment may also be carried out in this case, but when the abscess in the bone becomes too large, surgery to remove the abscess should be performed, or the tooth may have to be extracted. When root canal treatment that removes the pulp rather than protects the pulp cells is performed, the feeling of mastication, as with natural teeth, is not felt, and discomfort due to a foreign body sensation is inevitable. Regarding the immune response within the tooth, it is known that autophagy of odontoblasts is associated with the differentiation of new odontoblasts, cell viability, and protection from cell death. From the perspective of protection and adaptation of odontoblasts, autophagy of odontoblasts is expected to provide a beneficial effect on the survival of both pulp cells and new odontoblasts, but since odontoblasts are inevitably removed in the conventional root canal treatment method, the beneficial effect of odontoblasts is difficult to expect.

Against this background, the present inventors have made extensive research efforts to develop a preparation capable of more effectively treating dental caries and, as a result, developed a novel use of a peptide for promoting the regeneration of dentin or dental pulp tissue in preventing, ameliorating, or treating dental caries, thereby completing the present invention.

### [Disclosure]

### [Technical Problem]

One object of the present invention is to provide a use for ameliorating or treating dental caries, including root caries, dental caries of a deciduous tooth about to fall out, and dental caries of an erupting tooth, and pain caused thereby.

Another object of the present invention is to provide an effect for ameliorating or treating dental caries by increasing the healing ability of dental pulp damaged by dental caries and reducing the expression of inflammatory factors to prevent progression to irreversible pain.

Still, another object of the present invention is to provide a use for ameliorating or treating dental caries by increasing the autophagy of odontoblasts in an environment where the dental pulp is damaged by dental caries, thereby protecting the dental pulp and reactivating odontoblasts.

The objects of the present invention are not limited to those mentioned above, and other objects not mentioned will be clearly understood by those skilled in the art to which the present invention pertains from the description below.

### [Technical Solution]

As a result of conducting various studies, the present inventors developed a preparation for preventing, ameliorating, and treating dental caries, including a peptide consisting of 10 amino acids and a use thereof.

It was confirmed that the peptide is capable of increasing the expression levels of Dspp, Dmp1, and Nestin genes, which are odontoblast differentiation marker genes, and thus exhibits an effect of promoting dentin regeneration, while not exhibiting any cytotoxicity to the cells of the pulp tissue.

In addition, it was confirmed that the peptide forms a dentin-pulp-like tissue the form that is most similar to the *in vivo* dentin-pulp tissue, increases the level of collagen formation, and increases the expression level of dentin sialoprotein (DSP), which is an odontoblast-specific differentiation marker gene.

As an embodiment to achieve the above-mentioned objects, the present invention provides a pharmaceutical composition for treating dental caries, including a peptide consisting of an amino acid sequence of General Formula 1 below:

K-Y-R1-R2-R3-R4-R5-R6-R7-R8 (General Formula 1)

In General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).

The term "dentin" used herein refers to a yellowish-white hard tissue that makes up most of a tooth. Since the dentin is covered with enamel at the crown and cementum at the root, it is not exposed to the surface of a tooth. However, as the enamel wears away with age, the dentin may be exposed at the tip of the crown or the occlusal surface. The dentin is a type of bone tissue, but it is distinguished from general bone tissue in that the main body of the cells that make up the dentin is in the pulp, and only their protrusions extend into the dentin.

The term "dental pulp" used herein, also referred to as "pulp," refers to the soft connective tissue that fills the pulp cavity inside a tooth. Anatomically, it may also refer to the region in which nerves and blood vessels are abundantly distributed and which reaches the surface layer of the dentin.

The peptide included in the preparation provided by the present invention can increase the expression levels of Dspp, Dmp1, and Nestin genes, which are odontoblast differentiation marker genes, without exhibiting cytotoxicity, and when the peptide is transplanted *in vivo* together with dental pulp tissue cells, the dental pulp tissue cells can exhibit the characteristic of forming dentin/pulp tissue-like tissue.

With regard to the above peptide included in the preparation provided by the present invention, variant peptides having a sequence that differs by one or more amino acid residues from the amino acid sequence constituting the above peptide are also included in the scope of the peptide provided by the present invention, as long as they may exhibit a dental caries treatment effect.

In general, amino acid exchanges in proteins and polypeptides that do not alter the overall activity of the molecule are well known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In addition, peptides whose structural stability against heat, pH, etc., or whose ability to promote the regeneration of dentin or pulp tissue is increased by mutations or modifications in the amino acid sequence may be included.

For example, even when glutamine, an acidic amino acid located at position 3 of the peptide of SEQ ID NO: 1 provided by the present invention, is replaced with lysine or arginine, which are basic amino acids, the effect of the peptide provided by the present invention may be exhibited as is; even when arginine, a basic amino acid located at position 4 or 5 of the peptide of SEQ ID NO: 1, is replaced with glutamine, which is an acidic amino acid, or lysine, which is a basic amino acid, the effect of the peptide provided by the present invention may be exhibited as is; even when lysine, a basic amino acid located at position 6, 7 or 9 of the peptide of SEQ ID NO: 1, is replaced with arginine, which is a basic amino acid, or tyrosine, which is an aromatic amino acid, the effect of the peptide provided by the present invention may be exhibited as is; even when asparagine, an acidic amino acid located at position 8 of the peptide of SEQ ID NO: 1, is replaced with serine, which is a neutral amino acid, the effect of the peptide provided by the present invention may be exhibited as is; and even when tyrosine, an aromatic amino acid located at position 10 of the peptide of SEQ ID NO: 1, is replaced with lysine, a basic amino acid, the effect of the peptide provided by the present invention can be exhibited as is.

In this way, even when an acidic amino acid, basic amino acid, or aromatic amino acid constituting the peptide of the present invention is replaced with another acidic amino acid, basic amino acid, neutral amino acid, or aromatic amino acid, the effect of the peptide provided by the present invention may be exhibited as is, so a variant peptide having a sequence that differs by one or more amino acid residues from the amino acid sequence constituting the peptide of the present invention may also be included in the scope of the peptide provided by the present invention.

In addition, since the peptide included in the preparation of the present invention may exhibit the effect of the peptide provided by the present invention as is, even when it has a form in which any amino acid is added to its N-terminus or C-terminus, the peptide is included in the scope of the peptide provided by the present invention. As an example, it may be in a form in which 1 to 300 amino acids are added to the N-terminus or C-terminus of the peptide, as another example, it may be in a form in which 1 to 100 amino acids are added to the N-terminus or C-terminus of the peptide, and as still another example, it may be in a form in which 1 to 24 amino acids are added to the N-terminus or C-terminus of the peptide.

The 96 peptides corresponding to General Formula 1 provided in the present invention have a significantly increased mRNA level of the Dspp gene, which is an odontoblast differentiation marker, in mouse dental papilla cell (MDPC)-23 cells treated with the 96 peptides, compared to the mRNA level of the Dspp gene measured in MDPC-23 cells (control group) that were not treated with the peptides.

As reported so far, it is known that when the mRNA expression level of DSPP is increased, odontoblast differentiation and dentin regeneration are promoted, so it can be seen that the 96 peptides that exhibit the effect of increasing the mRNA level of the Dspp gene exhibit the effect of promoting odontoblast differentiation and dentin regeneration (Taduru Sreenath et al., the Journal of Biological Chemistry, 278, No. 27, Issue of July 4, pp. 24874-24880, 2003; William T. Butler et al., Connective Tissue Research, 44 (Suppl. 1): 171-178, 2003).

Another aspect of the present invention is that it provides a polynucleotide encoding of the peptide.

The polynucleotide may be mutated by substitution, deletion, insertion, or a combination thereof of one or more bases. When a nucleotide sequence is chemically synthesized, a synthetic method widely known in the art, for example, a method described in the literature (Engels and Uhlmann, Angew Chem IntEd Engl., 37:73-127, 1988), may be used, and it may be synthesized using triester, phosphite, phosphoramidite, and H-phosphate methods, PCR and other auto primer methods, oligonucleotide synthesis on a solid support, and the like. For example, a polynucleotide encoding a peptide of the present invention may include a base sequence of SEQ ID NO: 4.

As another aspect, the present invention provides an expression vector including the polynucleotide, a transformant including the expression vector, and a method of preparing the peptide using the transformant.

The term "expression vector" used herein refers to a recombinant vector capable of expressing a target peptide in a target host cell and means a genetic construct including essential regulatory elements operably linked to allow a gene insert to be expressed. The expression vector includes expression regulatory elements such as an initiation codon, a stop codon, a promoter, and an operator, and the initiation codon and the stop codon are generally considered to be part of a nucleotide sequence encoding a polypeptide and must be functional in an individual when the genetic construct is administered and must be in-frame with a coding sequence. The promoter of the vector may be constitutive or inducible.

The term "operably linked" used herein refers to a state in which a nucleic acid expression regulatory sequence and a nucleic acid sequence encoding a target protein or RNA are functionally linked to perform a general function. For example, a promoter and a nucleic acid sequence encoding a protein or RNA may be operably linked to affect the expression of a coding sequence. An operably linked expression vector may be produced using a genetic recombination technique well known in the art, and site-specific DNA cleavage and linkage may be performed using enzymes generally known in the art.

**In** addition, the expression vector may include a signal sequence for releasing the peptide to facilitate isolation of the peptide from a cell culture medium. Specific initiation signals may also be required for efficient translation of an inserted nucleic acid sequence. These signals include the ATG start codon and adjacent sequences. In some cases, an exogenous translational control signal must be provided, which may include the ATG start codon. These exogenous translational control signals and start codons may be from a variety of natural and synthetic sources. Expression efficiency may be increased by the introduction of appropriate transcriptional or translational enhancing factors.

In addition, the expression vector may further include a protein tag that may be optionally removed using an endopeptidase to facilitate the detection of the peptide.

The term "tag" used herein refer to a molecule exhibiting a quantifiable activity or characteristic and may be a fluorescent molecule, including a chemical fluorophore such as fluorescein, a polypeptide fluorophore such as a green fluorescent protein (GFP) or a related protein; or an epitope tag such as a Myc tag, a Flag tag, a histidine tag, a leucine tag, an IgG tag, or a straptavidin tag. In particular, when an epitope tag is used, a peptide tag may be used, preferably consisting of six or more amino acid residues, and more preferably consisting of 8 to 50 amino acid residues.

In the present invention, the expression vector may include a nucleotide sequence encoding the above-described peptide for promoting the regeneration of dentin or pulp tissue and treating dentin hypersensitivity of the present invention, and a vector used at this time is not particularly limited, as long as it is capable of producing the peptide, but may preferably be a plasmid DNA, phage DNA, and the like, and more preferably a commercially developed plasmid (pUC18, pBAD, pIDTSAMRT-AMP, etc.), an E. coli-derived plasmid (pYG601BR322, pBR325, pUC118, pUC119, etc.), a *Bacillus subtilis*-derived plasmid (pUB110, pTP5, etc.), a yeast-derived plasmid (YEp13, YEp24, YCp50, etc.), a phage DNA (Charon4A, Charon21A, EMBL3, EMBL4, λgt10, λgt11, λZAP, etc.), an animal virus vector (retrovirus, adenovirus, vaccinia virus, etc.), or an insect virus vector (baculovirus, etc.). Since the protein's expression level, modification, and the like vary depending on the host cell, a host cell that is most suitable for the purpose may be selected and used.

The transformant provided by the present invention may be produced by introducing the expression vector provided by the present invention into a host and transforming the same and may be used to produce the peptide by expressing the polynucleotide included in the expression vector. The transformation may be performed by various methods, and the method is not particularly limited, as long as it is capable of producing the peptide, but a CaCl₂ precipitation method, the Hanahan method, in which efficiency is increased by using a reducing substance called dimethyl sulfoxide (DMSO) in the CaCl₂ precipitation method, electroporation, the calcium phosphate precipitation method, a protoplast fusion method, a stirring method using silicon carbide fibers, an *Agrobacterium*-mediated transformation method, a transformation method using polyethylene glycol (PEG), and a dextran sulfate, lipofectamine, and drying/inhibition-mediated transformation method may be used. In addition, the host used for producing the transformant is also not particularly limited, as long as it is capable of producing the peptide, and it may be a bacterial cell such as *Escherichia coli, Streptomyces,* or *Salmonella typhimurium*; a yeast cell such as *Saccharomyces cerevisiae* or *Schizosaccharomyces pombe*; a fungal cell such as *Pichia pastoris*; an insect cell such as *Drosophila* or *Spodoptera Sf9* cells; an animal cell such as Chinese hamster ovary (CHO), COS, NS0, 293, or Bowes melanoma cells; or a plant cell.

The transformant may also be used in a method of producing a peptide for promoting the regeneration of dentin or pulp tissue and treating dentin hypersensitivity of the present invention. Specifically, the method of producing a peptide for promoting the regeneration of dentin or pulp tissue and treating dentin hypersensitivity of the present invention may include (a) culturing the transformant to obtain a culture and (b) recovering the peptide of the present invention from the culture.

The term "culture" used herein refers to a method of growing microorganisms under appropriately artificially controlled environmental conditions. In the present invention, the method of culturing the transformant may be performed using a method widely known in the art. Specifically, the culturing is not particularly limited as long as it is capable of expressing and producing the peptide for promoting the regeneration of dentin or pulp tissue and treating dentin hypersensitivity of the present invention, but the culture may be performed in a continuous manner in a batch process or a fed-batch or repeated fed-batch process.

The medium used for culture should be a conventional medium containing a suitable carbon source, nitrogen source, amino acids, vitamins, and the like, and satisfy the requirements of a specific strain in an appropriate manner while the temperature, pH, and the like are controlled under aerobic conditions. The carbon sources that may be used include mixed sugars of glucose and xylose as the main carbon source, as well as sugars and carbohydrates such as sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture. As the nitrogen sources that may be used, inorganic nitrogen sources such as ammonia, ammonium sulfate, ammonium chloride, ammonium acetate, ammonium phosphate, ammonium carbonate, and ammonium nitrate; organic nitrogen sources such as amino acids such as glutamic acid, methionine, and glutamine, and peptone, NZ-amine, meat extracts, yeast extracts, malt extracts, corn steep liquor, casein hydrolysate, fish or decomposition products thereof, defatted soybean cake or decomposition product thereof may be used. These nitrogen sources may be used alone or in combination. The medium may contain monopotassium phosphate, dipotassium phosphate, and corresponding sodium-containing salts as phosphorus sources. Phosphorus sources that may be used include potassium dihydrogen phosphate or dipotassium hydrogen phosphate, or corresponding sodium-containing salts. In addition, inorganic compounds such as sodium chloride, calcium chloride, iron chloride, magnesium sulfate, iron sulfate, manganese sulfate, and calcium carbonate may be used. Lastly, in addition to the above substances, essential growth substances such as amino acids and vitamins may be used.

In addition, suitable precursors may be used in the culture medium. The above-described raw materials may be added to the culture in a batch, fed-batch, or continuous manner in an appropriate manner during the culture process, but the method is not particularly limited thereto. The pH of the culture may be adjusted by using basic compounds such as sodium hydroxide, potassium hydroxide, ammonia, or acidic compounds such as phosphoric acid or sulfuric acid in an appropriate manner.

In addition, antifoaming agents such as fatty acid polyglycol esters may be used to suppress foaming. Oxygen or an oxygen-containing gas (e.g., air) is injected into the culture to maintain aerobic conditions. The temperature of the culture is usually 27 °C to 37 °C, preferably 30 °C to 35 °C. The culturing is continued until the maximum production of the peptide is obtained. This purpose is usually achieved in 10 to 100 hours.

In addition, the recovering of the peptide from the culture may be performed by a method known in the art. Specifically, the recovery method is not particularly limited, as long as it may be used to recover the produced peptide, but preferably, methods such as centrifugation, filtration, extraction, spraying, drying, evaporation, precipitation, crystallization, electrophoresis, differential dissolution (e.g., ammonium sulfate precipitation), chromatography (e.g., ion exchange, affinity, hydrophobicity, and size exclusion) may be used.

The pharmaceutical composition of the present invention may promote the formation of dentin/pulp tissue-like tissue by pulp tissue cells and thus may be used to treat dental caries by a method of restoring dentin or pulp tissue.

The peptide included in the pharmaceutical composition may be used in the form of a single peptide, may be used in the form of a polypeptide in which the peptide is linked by repeating it two or more times, or may be used in the form of a complex in which a drug exhibiting an antibiotic effect against dental caries-causing bacteria is bound to the N-terminus or C-terminus of the peptide.

The term "dental caries" used herein may refer to an infectious disease caused by bacteria living on the tooth surface.

The term "prevention" used herein refers to all acts of inhibiting or delaying the occurrence of dental caries by administering a pharmaceutical composition for preventing or treating dental caries, including the peptide of the present invention.

The term "treatment" of the present invention refers to all acts of promoting the regeneration of dentin or pulp tissue by administering a pharmaceutical composition including the peptide of the present invention as an active ingredient to an individual in need of dental caries treatment, thereby performing treatment of dental caries.

The pharmaceutical composition of the present invention may be prepared in the form of a pharmaceutical composition for treating dental caries, which further includes a suitable carrier (natural or non-natural carrier), excipient, or diluent commonly used in the preparation of pharmaceutical compositions as well the peptide. Specifically, the pharmaceutical composition may be formulated and used in the form of a sterile injectable solution that may be administered to a region where dental caries has been induced according to a conventional method. In the present invention, examples of the carriers, excipients, and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and collagen. When the peptide of the present invention is formulated, it may be prepared using diluents or excipients such as commonly used fillers, bulking agents, binders, wetting agents, disintegrating agents, and surfactants. In particular, sterilized aqueous solutions, non-aqueous solvents, suspensions, emulsions, lyophilized preparations, suppositories, and ointments (e.g., pulp liners, etc.) may be included. As a non-aqueous solvent and a suspension, propylene glycol, polyethylene glycol, vegetable oils such as olive oil, and injectable esters such as ethyl oleate may be used. As a base for suppositories, Witepsol, Macrogol, Tween 61, cacao oil, laurel oil, glycerol, gelatin, or the like may be used.

The amount of the peptide included in the pharmaceutical composition of the present invention is not particularly limited, but it may be included in an amount of 0.0001% to 50% by weight, more preferably 0.01% to 20% by weight, based on the total weight of the final composition.

The pharmaceutical composition of the present invention may be administered in a pharmaceutically effective amount, and the term "pharmaceutically effective amount" used herein refers to an amount that is sufficient to treat or prevent disease with a reasonable benefit/risk ratio applicable to medical treatment or prevention and an effective dose level may be determined based on factors including the severity of a disease, drug activity, the patient's age, weight, health conditions, sex, the patient's sensitivity to the drug, administration time, administration route, and excretion rate of the used composition of the present invention, treatment duration, drugs that are mixed or concurrently used with the composition of the present invention, and other factors well known in the medical field. The pharmaceutical composition of the present invention may be administered alone or in combination with a known pharmaceutical composition for treating dental caries. It is important to administer an amount that may achieve the maximum effect with the minimum amount without side effects by considering all of the above factors.

The dosage of the pharmaceutical composition of the present invention may be determined by a person skilled in the art in consideration of the intended use, the severity of the disease, the patient's age, weight, sex, medical history, or the type of substance used as an active ingredient. For example, the pharmaceutical composition of the present invention may be administered to an adult in an amount of about 0.1 ng/kg to about 100 mg/kg, preferably 1 ng/kg to about 10 mg/kg, and the frequency of administration of the composition of the present invention is not particularly limited, but it may be administered once a day or administered several times in divided doses. The above dosage does not limit the scope of the present invention in any way.

Another aspect of the present invention is that it provides a method of treating dental caries, including administering the pharmaceutical composition in a pharmaceutically effective amount to an individual other than a human suffering from dental caries.

The term "individual" used herein may include, without limitation, mammals, including rats, livestock, and the like, that require treatment for dental caries but may exclude humans from among the individuals suffering from the disease.

The administration route of the pharmaceutical composition for treating dental caries of the present invention may be administered via any general route as long as it reaches the target tissue. The present invention's pharmaceutical composition is not limited but may be administered via a route such as intraoral administration or intraoral injection, depending on the purpose.

As another embodiment, the present invention provides a quasi-drug composition for preventing or ameliorating dental caries, including the peptide.

The term "amelioration" herein refers to all acts of at least reducing a parameter related to the condition being treated, such as the degree of symptoms.

In the present invention, the amelioration may be interpreted to mean all acts of ameliorating the symptoms of dental caries or providing benefits by promoting the regeneration of dentin or pulp tissue by administering the pharmaceutical composition, including the peptide of the present invention as an active ingredient to an individual in need of treatment of dental caries.

The term "quasi-drug" used herein refers to products used for diagnosing, curing, ameliorating, alleviating, treating, or preventing diseases of humans or animals and have a milder effect than drugs. For example, according to the Pharmaceutical Affairs Act, quasi-drugs are products other than those used for the purpose of drugs and include fiber and rubber products used for the treatment or prevention of diseases of humans or animals, things that have a mild or no direct effect on the human body, things that are not instruments or machines and similar thereto, and sterilizers and insecticides for preventing infectious diseases.

In the present invention, the type or formulation of the pharmaceutical composition, including the peptide, is not particularly limited but may be, for example, an oral disinfectant, oral cleaning product, toothpaste, dental floss, oral ointment, and the like.

As another aspect, the present invention provides a health functional food composition for preventing or ameliorating dental caries, including peptides.

The term "food" used herein includes meat, sausages, bread, chocolates, candies, snacks, confectioneries, pizza, ramen, other noodles, gum, dairy products including ice cream, various soups, beverages, tea, drinks, alcoholic beverages, vitamin complexes, health functional foods, and health foods, and includes all foods in a conventional sense.

The above functional health food is the same term as food for special health use (FoSHU) and refers to food with high medical and healthcare effects that are processed to efficiently exhibit a bioregulatory function in addition to nutritional supply. Here, "functionality" refers to regulating nutrients for the structure and function of the human body or obtaining a useful effect for health purposes such as physiological effects. The food of the present invention may be produced by a method commonly used in art and may be produced by adding raw materials and ingredients commonly added to art during the production process. In addition, the formulation of the food may be prepared without limitation as long as it is a formulation recognized as a food. The food composition of the present invention may be prepared in various forms of formulations, and unlike general drugs, it has the advantage of not having side effects that may occur when taking drugs for a long period of time because food is used as a raw material, and it is highly portable, so the food of the present invention may be consumed as a supplement to enhance the effect of preventing or ameliorating dental caries.

Health food refers to food that has a more active health maintenance or promotion effect than general food, and health supplement food refers to food for the purpose of health supplementation. In some cases, the terms health functional food, health food, and health supplement food may be used interchangeably.

Specifically, health-functional food refers to a food product which is produced by adding the peptide of the present invention to food materials such as beverages, tea, spices, gum, and confectioneries or produced in the form of capsules, powder, suspensions, or the like, and which has a specific health effect when consumed. However, unlike general drugs, it has the advantage of not having side effects that may occur when taking drugs for a long period of time because food is used as a raw material.

Since the food composition of the present invention may be routinely consumed, it can be expected to be highly effective in preventing or ameliorating dental caries and thus can be used very usefully.

The above food composition may further include a physiologically acceptable carrier, and the type of carrier is not particularly limited. Any carrier commonly used in art may be used.

In addition, the food composition may include additional ingredients commonly used in food compositions to improve odor, taste, appearance, and the like. For example, it may include vitamins A, C, D, E, B1, B2, B6, B12, niacin, biotin, folate, pantothenic acid, and the like. In addition, it may include minerals such as zinc (Zn), iron (Fe), calcium (Ca), chromium (Cr), magnesium (Mg), manganese (Mn), copper (Cu), and chromium (Cr). In addition, it may include amino acids such as lysine, tryptophan, cysteine, and valine.

In addition, the food composition may include food additives such as preservatives (potassium sorbate, sodium benzoate, salicylic acid, sodium dehydroacetate, etc.), bactericides (bleaching powder and highly bleaching powder, sodium hypochlorite, etc.), antioxidants (butylated hydroxyanisole (BHA), butylated hydroxytoluene (BHT), etc.), dyes (tar color, etc.), colorants (sodium nitrite, etc.), bleaching agents (sodium sulfite), seasonings (monosodium glutamate (MSG), etc.), sweeteners (dulcin, cyclamate, saccharin, sodium, etc.), flavoring agents (vanillin, lactones, etc.), leavening agents (alum, D-potassium hydrogen tartrate, etc.), fortifiers, emulsifiers, thickeners (gelling agents), film-forming agents, gum bases, anti-foaming agents, solvents, and improvers. The above additives may be selected and used in an appropriate amount depending on the type of food.

The peptide of the present invention may be added as is or used together with other food or food ingredients and may be used appropriately according to a conventional method. The mixing amount of an active ingredient may be appropriately determined according to its purpose of use (prevention, health, or therapeutic treatment). In general, when a food or beverage is produced, the food composition of the present invention may be added to the food or beverage in an amount of 50 parts by weight or less, specifically 20 parts by weight or less. However, in case of long-term intake for the purpose of health and hygiene, an amount below the above range may be included, and since there is no problem in terms of safety, the active ingredient may be used in an amount above the range.

As an example, the food composition of the present invention may be used as a health beverage composition, and in this case, various flavoring agents or, natural carbohydrates, or the like may be contained as additional ingredients, like a common beverage. The above-mentioned natural carbohydrates may include monosaccharides such as glucose and fructose; disaccharides such as maltose and sucrose; polysaccharides such as dextrin, and cyclodextrin; and sugar alcohols such as xylitol, sorbitol, and erythritol. The sweeteners may include natural sweeteners such as thaumatin and stevia extract and synthetic sweeteners such as saccharin and aspartame. The proportion of natural carbohydrates may be generally about 0.01 to 0.04 g, specifically about 0.02 to 0.03 g per 100 mL of the health beverage composition of the present invention.

In addition to the above, the health beverage composition may contain various nutrients, vitamins, electrolytes, flavoring agents, stains, pectic acid, salts of pectic acid, alginic acid, salts of alginic acid, organic acids, protective colloid thickeners, pH regulators, stabilizers, preservatives, glycerin, alcohols, or carbonating agents. In addition, it may contain fruit pulp for producing natural fruit juice, fruit juice beverages, or vegetable beverages. These ingredients may be used independently or in combination. The proportion of these additives is not particularly important but is generally selected in the range of 0.01 to 0.1 parts by weight per 100 parts by weight of the health beverage composition of the present invention.

The food composition of the present invention may include the peptide of the present invention in various percentages by weight as long as it is capable of exhibiting the effect of preventing or ameliorating dental caries, but specifically, the peptide of the present invention may be included in an amount of 0.00001% to 100% by weight or 0.01% to 80% by weight based on the total weight of the food composition, but is not limited thereto.

### [Advantageous Effects]

The present invention can provide use for ameliorating or treating dental caries, including root caries, dental caries of a deciduous tooth about to fall out, and dental caries of an erupting tooth and pain caused thereby.

Another object of the present invention is to provide an effect for ameliorating or treating dental caries by increasing the healing ability of dental pulp damaged by dental caries and reducing the expression of inflammatory factors to prevent progression to irreversible pain.

The present invention can provide use for ameliorating or treating dental caries by increasing the autophagy of odontoblasts in an environment where the dental pulp is damaged by dental caries, thereby protecting the dental pulp and reactivating odontoblasts.

The effects of the present invention are not limited to those mentioned above, and other effects not mentioned will be clearly understood by those skilled in the art to which the present invention pertains from the description below.

### [Description of Drawings]

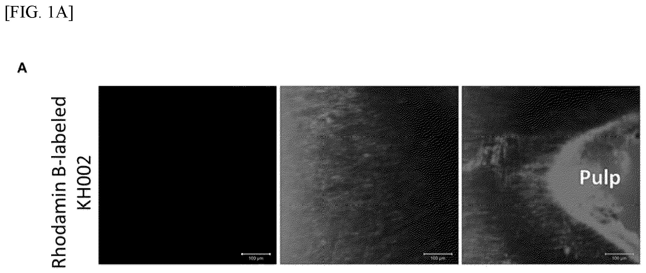

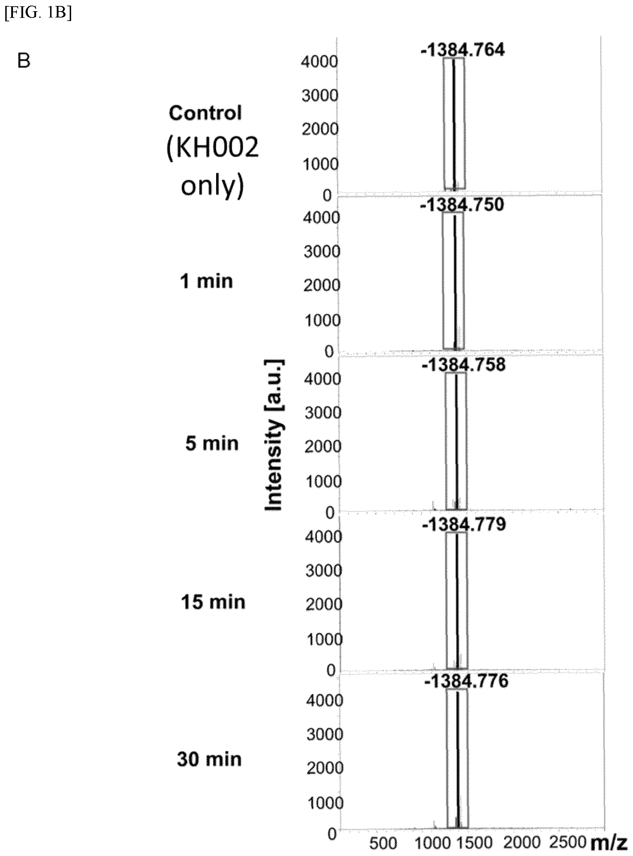

FIGS. 1A and 1B show the results of observing the permeability and stability of a peptide (SEQ ID NO: 96) included in pharmaceutical compositions according to an embodiment of the present invention in a dental caries environment, respectively. FIG. 1A shows the results of extracting a tooth from a human and exhibiting signs of dentin dental caries (stages 2 to 3 of dental caries), applying a peptide conjugated to rhodamine, a fluorescent dye, to the tooth, and after about one minute, fixing the tooth with 4% paraformaldehyde, and cutting a tooth slice in the sagittal direction using a digital low-speed diamond cutter to a thickness of 0.1 mm and observing the tooth slice under a confocal microscope. FIG. 1B shows the results of confirming the stability of the peptide in the pharmaceutical composition in an environment in which *S. Mutans,* a causative bacterium of dental caries, is present through matrix-assisted laser desorption/ionization time-of-flight mass (MALDI-TOF) analysis.

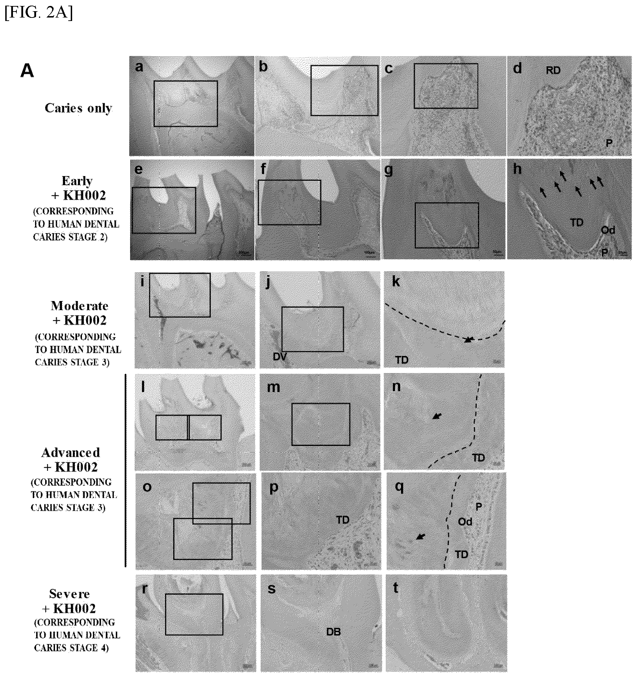

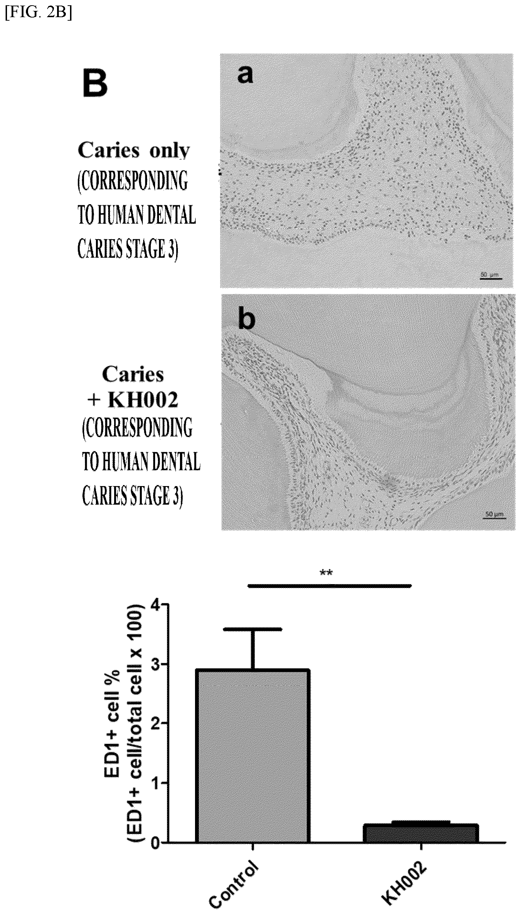

FIG. 2A shows the results of treating a dental caries rat model with a pharmaceutical composition according to an embodiment of the present invention at each stage of dental caries and FIG. 2B shows the results of comparing the number of inflammatory cells when a tooth affected by dental caries was treated with a pharmaceutical composition according to an embodiment of the present invention in the dental caries rat model.

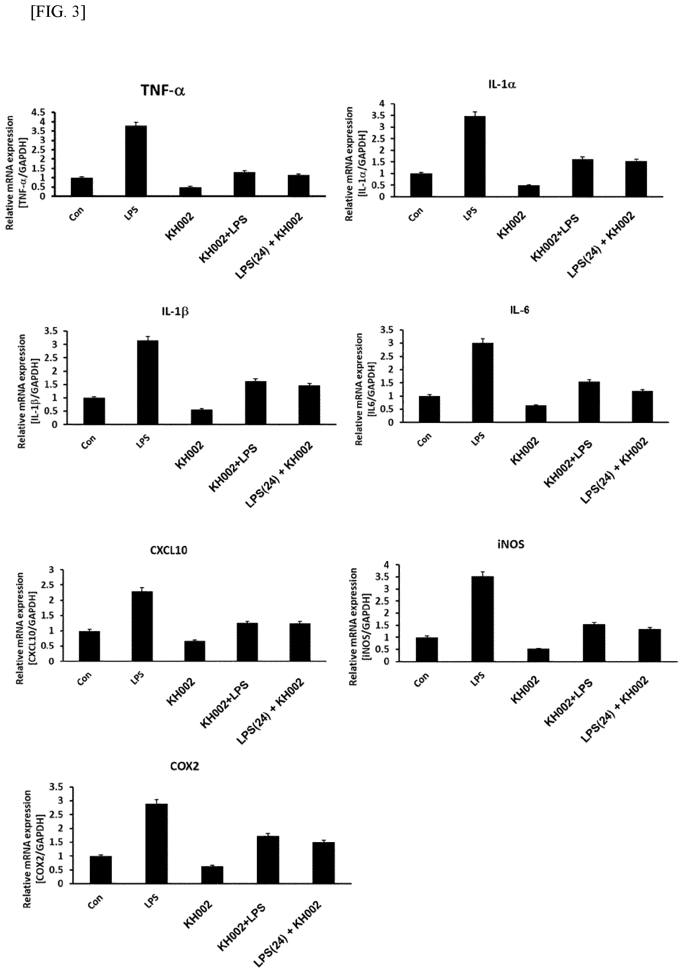

FIG. 3 shows a cell experiment simulating pulp inflammation caused by dental caries corresponding to stage 4, and shows a graph illustrating the effect of treating human dental pulp cells with a pharmaceutical composition according to an embodiment of the present invention under an inflammatory environment on the expression of genes related to inflammatory factors.

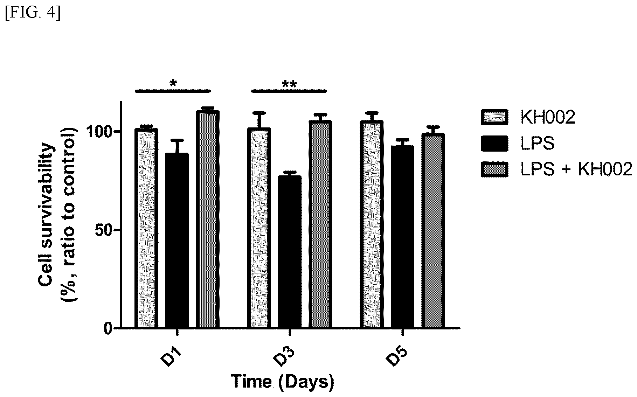

FIG. 4 shows a cell experiment simulating a situation where pulp inflammation caused by dental caries corresponding to stage 4 occurs and shows a graph illustrating the effect of treating human dental pulp cells with a pharmaceutical composition according to an embodiment of the present invention under an inflammatory environment on the viability of dental pulp cells.

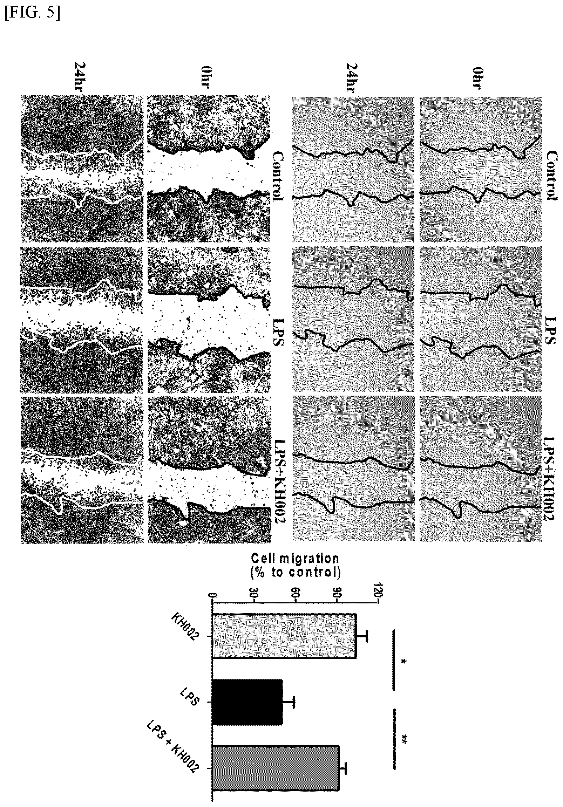

FIG. 5 shows a cell experiment simulating a situation where pulp inflammation caused by dental caries corresponding to stage 4 occurs and illustrates the effect of treating damaged human pulp cells with a pharmaceutical composition according to an embodiment of the present invention under an inflammatory environment on the recovery of the pulp cells.

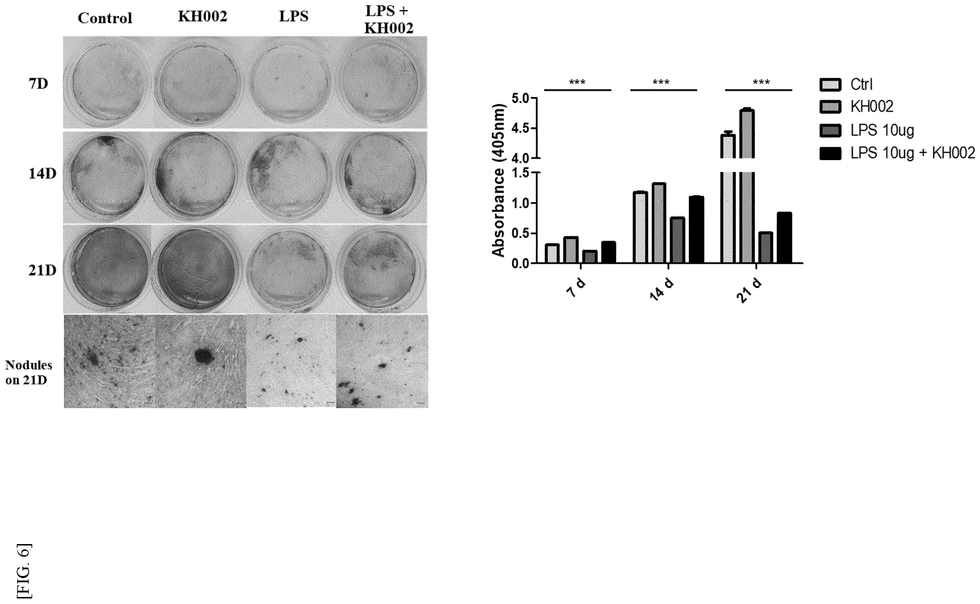

FIG. 6 shows a cell experiment simulating a situation when pulp inflammation caused by dental caries corresponding to stage 4 occurs and illustrates the results of examining the effect on the mineralization ability of human pulp cells.

### [Modes of the Invention]

The purpose and effect of the present invention and the technical configurations for achieving them will become clearer with reference to the embodiments described in detail below with the attached drawings. In describing the present invention, when it is judged that a specific description of a known function or configuration may unnecessarily obscure the gist of the present invention, the detailed description thereof will be omitted. In addition, the terms described below are terms defined in consideration of the functions in the present invention, and these may vary depending on the intention or custom of the user or operator.

However, the present invention is not limited to the embodiments disclosed below, but can be implemented in various different forms. These embodiments are presented only to ensure that the disclosure of the present invention is complete and to fully inform those skilled in the art to which the present invention pertains of the scope of the invention and the present invention is defined only by the scope of the claims. Therefore, the definition should be based on the contents of the present specification.

Hereinafter, the present invention will be described in more detail through examples. However, these examples are intended to illustrate the present invention, and the scope of the present invention is not limited to these examples.

### Example 1: Synthesis of peptide

The present inventors synthesized a peptide (SEQ ID NO: 1) exhibiting a dentin or pulp tissue regeneration promoting effect by the 9-fluorenylmethyloxycarbonyl (Fmoc) method and synthesized peptides of each group by replacing the amino acids of the synthesized peptide (Tables 1 to 12).
N-KYQRRKKNKY-C (SEQ ID NO: 1)

First, the peptides of Group 1 were synthesized by replacing the amino acids 5 to 7 of the peptide of SEQ ID NO: 1 with lysine or arginine and included the peptide of SEQ ID NO: 1 as well (Table 1).

**[Table 1]**

| Peptides of Group 1 | |
|---|---|
| SEQ ID NO. | Amino acid sequence (N-C) |
| 1 | KYQRRKKNKY |
| 2 | KYQRRKRNKY |
| 3 | KYQRRRKNKY |
| 4 | KYQRRRRNKY |
| 5 | KYQRKKKNKY |
| 6 | KYQRKRKNKY |
| 7 | KYQRKKRNKY |
| 8 | KYQRKRRNKY |

Next, the peptides of Group 2 were synthesized by replacing amino acids 5 to 7 of the SEQ ID NO: 1 peptide with lysine or arginine and replacing amino acid 8 with serine (Table 2).

**[Table 2]**

| Peptides of Group 2 | |
|---|---|
| SEQ ID NO. | Amino acid sequence (N-C) |
| 9 | KYQRRKKSKY |
| 10 | KYQRRKRSKY |
| 11 | KYQRRRKSKY |
| 12 | KYQRRRRSKY |
| 13 | KYQRKKKSKY |
| 14 | KYQRKRKSKY |
| 15 | KYQRKKRSKY |
| 16 | KYQRKRRSKY |

Next, the peptides of Group 3 were synthesized by replacing amino acids 5 to 7 of the SEQ ID NO: 1 peptide with lysine or arginine and replacing amino acid 9 with tyrosine (Table 3).

**[Table 3]**

| Peptides of Group 3 | |
|---|---|
| SEQ ID NO. | Amino acid sequence (N-C) |
| 17 | KYQRRKKNYK |
| 18 | KYQRRKRNYK |
| 19 | KYQRRRKNYK |
| 20 | KYQRRRRNYK |
| 21 | KYQRKKKNYK |
| 22 | KYQRKRKNYK |
| 23 | KYQRKKRNYK |
| 24 | KYQRKRRNYK |

Next, the peptides of Group 4 were synthesized by replacing amino acids 5 to 7 of the peptide of SEQ ID NO: 1 with lysine or arginine, replacing amino acid 8 with serine, replacing amino acid 9 with tyrosine, and replacing amino acid 10 with lysine (Table 4).

**[Table 4]**

| Peptides of Group 4 | |
|---|---|
| SEQ ID NO. | Amino acid sequence (N-C) |
| 25 | KYQRRKKSYK |
| 26 | KYQRRKRSYK |
| 27 | KYQRRRKSYK |
| 28 | KYQRRRRSYK |
| 29 | KYQRKKKSYK |
| 30 | KYQRKRKSYK |
| 31 | KYQRKKRSYK |
| 32 | KYQRKRRSYK |

Next, the peptides of Group 5 were synthesized by replacing amino acid 3 of the SEQ ID NO: 1 peptide with arginine, amino acid 4 with glutamine, and amino acids 5 to 7 with lysine or arginine (Table 5).

**[Table 5]**

| Peptides of Group 5 | |
|---|---|
| SEQ ID NO. | Amino acid sequence (N-C) |
| 33 | KYRQRKKNKY |
| 34 | KYRQRKRNKY |
| 35 | KYRQRRKNKY |
| 36 | KYRQRRRNKY |
| 37 | KYRQKKKNKY |
| 38 | KYRQKRKNKY |
| 39 | KYRQKKRNKY |
| 40 | KYRQKRRNKY |

Next, the peptides of Group 6 were synthesized by replacing amino acid 3 of the peptide of SEQ ID NO: 1 with arginine, replacing amino acid 4 with glutamine, replacing amino acids 5 to 7 with lysine or arginine, and replacing amino acid 8 with serine (Table 6).

**[Table 6]**

| Peptides of Group 6 | |
|---|---|
| SEQ ID NO. | Amino acid sequence (N-C) |
| 41 | KYRQRKKSKY |
| 42 | KYRQRKRSKY |
| 43 | KYRQRRKSKY |
| 44 | KYRQRRRSKY |
| 45 | KYRQKKKSKY |
| 46 | KYRQKRKSKY |
| 47 | KYRQKKRSKY |
| 48 | KYRQKRRSKY |

Next, the peptides of Group 7 were synthesized by replacing amino acid 3 of the peptide of SEQ ID NO: 1 with arginine, replacing amino acid 4 with glutamine, replacing amino acids 5 to 7 with lysine or arginine, replacing amino acid 9 with tyrosine, and replacing amino acid 10 with lysine (Table 7).

**[Table 7]**

| Peptides of Group 7 | |
|---|---|
| SEQ ID NO. | Amino acid sequence (N-C) |
| 49 | KYRQRKKNYK |
| 50 | KYRQRKRNYK |
| 51 | KYRQRRKNYK |
| 52 | KYRQRRRNYK |
| 53 | KYRQKKKNYK |
| 54 | KYRQKRKNYK |
| 55 | KYRQKKRNYK |
| 56 | KYRQKRRNYK |

Next, the peptides of Group 8 were synthesized by replacing amino acid 3 of the peptide of SEQ ID NO: 1 with arginine, replacing amino acid 4 with glutamine, replacing amino acids 5 to 7 with lysine or arginine, replacing amino acid 8 with serine, replacing amino acid 9 with tyrosine, and replacing amino acid 10 with lysine (Table 8).

**[Table 8]**

| Peptides of Group 8 | |
|---|---|
| SEQ ID NO. | Amino acid sequence (N-C) |
| 57 | KYRQRKKSYK |
| 58 | KYRQRKRSYK |
| 59 | KYRQRRKSYK |
| 60 | KYRQRRRSYK |
| 61 | KYRQKKKSYK |
| 62 | KYRQKRKSYK |
| 63 | KYRQKKRSYK |
| 64 | KYRQKRRSYK |

Next, the peptides of Group 9 were synthesized by replacing amino acid 3 of the SEQ ID NO: 1 peptide with lysine, amino acid 4 with glutamine, and amino acids 5 to 7 with lysine or arginine (Table 9).

**[Table 9]**

| Peptides of Group 9 | |
|---|---|
| SEQ ID NO. | Amino acid sequence (N-C) |
| 65 | KYKQRKKNKY |
| 66 | KYKQRKRNKY |
| 67 | KYKQRRKNKY |
| 68 | KYKQRRRNKY |
| 69 | KYKQKKKNKY |
| 70 | KYKQKRKNKY |
| 71 | KYKQKKRNKY |
| 72 | KYKQKRRNKY |

Next, the peptides of Group 10 were synthesized by replacing amino acid 3 of the peptide of SEQ ID NO: 1 with lysine, replacing amino acid 4 with glutamine, replacing amino acids 5 to 7 with lysine or arginine, and replacing amino acid 8 with serine (Table 10).

**[Table 10]**

| Peptides of Group 10 | |
|---|---|
| SEQ ID NO. | Amino acid sequence (N-C) |
| 73 | KYKQRKKSKY |
| 74 | KYKQRKRSKY |
| 75 | KYKQRRKSKY |
| 76 | KYKQRRRSKY |
| 77 | KYKQKKKSKY |
| 78 | KYKQKRKSKY |
| 79 | KYKQKKRSKY |
| 80 | KYKQKRRSKY |

Next, the peptides of Group 11 were synthesized by replacing amino acid 3 of the peptide of SEQ ID NO: 1 with lysine, replacing amino acid 4 with glutamine, replacing amino acids 5 to 7 with lysine or arginine, replacing amino acid 9 with tyrosine, and replacing amino acid 10 with lysine (Table 11).

**[Table 11]**

| Peptides of Group 11 | |
|---|---|
| SEQ ID NO. | Amino acid sequence (N-C) |
| 81 | KYKQRKKNYK |
| 82 | KYKQRKRNYK |
| 83 | KYKQRRKNYK |
| 84 | KYKQRRRNYK |
| 85 | KYKQKKKNYK |
| 86 | KYKQKRKNYK |
| 87 | KYKQKKRNYK |
| 88 | KYKQKRRNYK |

Lastly, the peptides of Group 12 were synthesized by replacing amino acid 3 of the peptide of SEQ ID NO: 1 with lysine, replacing amino acid 4 with glutamine, replacing amino acids 5 to 7 with lysine or arginine, replacing amino acid 8 with serine, replacing amino acid 9 with tyrosine, and replacing amino acid 10 with lysine (Table 12).

**[Table 12]**

| Peptides of Group 12 | |
|---|---|
| SEQ ID NO. | Amino acid sequence (N-C) |
| 89 | KYKQRKKSYK |
| 90 | KYKQRKRSYK |
| 91 | KYKQRRKSYK |
| 92 | KYKQRRRSYK |
| 93 | KYKQKKKSYK |
| 94 | KYKQKRKSYK |
| 95 | KYKQKKRSYK |
| 96 | KYKQKRRSYK |

### Example 2: Preparation of pharmaceutical composition

The method of preparing a pharmaceutical composition (KH002) according to an embodiment of the present invention is described below.

The pharmaceutical composition (KH002) was prepared by mixing about 1.5% carboxymethyl cellulose (Sigma-Aldrich, C5678). Carboxymethyl cellulose (Sigma-Aldrich, C5678) was mixed with distilled water in an amount of about 3%, stirred at 100 °C, and sterilized under pressurized conditions, and was prepared to achieve a final concentration of about 1.5% when mixed with the peptide having the SEQ ID NO: 96 (KH001) among the peptides prepared according to Example 1.

### Example 3: Permeability and stability of pharmaceutical composition in dental caries environment

### Example 3-1: Permeability of pharmaceutical composition in dental caries environment

A pharmaceutical composition (KH002) containing a peptide (KH001) conjugated with rhodamine, a fluorescent dye, was applied to an extracted human tooth with dentin dental caries (dental caries stage 2 to 3), and after about one minute, it was fixed with 4% paraformaldehyde. Then, a tooth slice was cut in the sagittal direction using a digital low-speed diamond cutter to a thickness of 0.1 mm and observed under a confocal microscope (FIG. 1A).

FIG. 1A shows a red-stained appearance, and it was confirmed that the peptide (KH001) ingredient included in the pharmaceutical composition, according to Example 2 penetrated well into the tooth affected by dental caries and reached the pulp.

### Example 3-2: Stability analysis of pharmaceutical composition in dental caries environment

### A. Analysis of the stability of peptide in the pharmaceutical composition provided by Example 2

The standard strain used in this experiment, *Streptococcus mutans* (*S.* mutans, KCOM 1054), was obtained from the Korean Collection of Oral Microbiology (KCOM). The cells were spread on a mitis salivarius-bacitracin (MSB; bacitracin concentration: 0.5 µg/mL) agar plate containing about 20% sucrose and cultured in a candle jar at about 37 °C for about 48 hours, and a single colony was cultured in a Tryptic Soy Broth (TSB; BD DIFCO Inc., USA) culture medium for about 24 hours and used in the experiment. The *S. mutans* strain was cultured until the absorbance (OD600) value reached 1.0, and 100 µl of the cultured strain and 100 µg of the pharmaceutical composition (KH002) in 1.5% carboxyl methyl cellulose were mixed. The stability of the peptide (KH001) included in the pharmaceutical composition (KH002) according to the reaction time with the strain was analyzed by MALDI-TOF analysis (Ultraflex III TOF/TOF (Bruker Daltonics Inc.)).

B. The composition of A was obtained at intervals of 1 minute, 5 minutes, 15 minutes, and 30 minutes and centrifuged at about 15,000 rpm for about 10 minutes, and the supernatants were collected.

C. 2 µL of each supernatant collected in B was mixed with 2 µL of substrate solution (10 mg/mL of α-cyano-4-hydroxycinnamic acid (CHCA) in 0.1% trifluoroacetic acid (TFA)/acetonitrile (ACN) (1:1, v/v)) and MALDI-TOF analysis was performed (Ultraflex III TOF/TOF (Bruker Daltonics Inc.)) (FIG. 1B).

Through FIG. 1B, it was confirmed that the peptide (KH001) in the pharmaceutical composition (KH002) provided by Example 2 was stably maintained in the environment of *S. Mutans,* a causative bacterium of dental caries.

### Example 4: Analysis of the results of treatment of dental caries by stage with pharmaceutical composition

### Example 4-1: Preparation of dental caries rat model by stage

FIG. 2A shows the stages of dentin caries in a dental caries rat model, divided into early, moderate, and advanced stages by 1/3 of the dentin thickness, where each stage was prepared by classifying the cases where the dentin above the pulp was completely affected by dental caries and the pulp was invaded as the severe stage, which was the most serious (n=4). In this classification, in comparison with the stages of human dental caries, the early stage is almost similar to stage 2 of dental caries, the moderate stage and the advanced stage are almost similar to stage 3 of dental caries, and the severe stage to is almost similar to stage 4 of dental caries.

### Example 4-2: Observation of results of treatment with pharmaceutical compositions in rat models

The control group was a group that was affected by dental caries but was not applied with the pharmaceutical composition (KH002) prepared according to Example 2. The tissue photographs below show the tissues of the rats to which 3 µg of the pharmaceutical composition (KH002) per tooth was applied at the early, moderate, advanced, and severe stages, and the rats were sacrificed four weeks later and fixed and decalcified, and their tissue slides were produced and stained by hematoxylin and eosin staining.

In the control group that was not treated with the pharmaceutical composition (KH002) prepared according to Example 2, liquefactive necrosis was observed inside the pulp, the palisade pattern of odontoblasts in the lower part affected by dental caries was damaged, and inflammatory cells were observed to have infiltrated into the pulp. In addition, in all groups treated with the pharmaceutical composition (KH002), the odontoblasts in the lower part affected by dental caries showed a palisade pattern, and stellate cells, which are a characteristic of healthy pulp, were observed throughout the pulp in the early and moderate stages, and it was confirmed that even in the severe state where all the dentin above the pulp was affected by dental caries, healthy pulp was maintained in the root area of the tooth.

When the pharmaceutical composition (KH002) was treated, it was observed that dentin was regenerated in all stages of dental caries, and even in the severely damaged tooth model, it was observed that a dentin bridge, which is formed as one of the defensive actions in dental caries, was formed above the preserved pulp.

### Example 4-3: Observation of inflammatory cells according to dental caries

The number of inflammatory cells was compared as a percentage of the total number of dental pulp cells (n=4 based on the number of individuals, n=6 based on the number of teeth). Three areas inside the dental pulp were randomly designated, and three researchers conducted the reading, and the researchers were blinded to ensure accurate reading.

FIG. 2A shows the results of treating a dental caries rat model with a pharmaceutical composition according to an embodiment of the present invention at each stage of dental caries. Referring to FIG 2A, it was confirmed that the proportion of inflammatory cells stained with ED1 was significantly reduced in the experimental group treated with the pharmaceutical composition (KH002) compared to the control group that was not treated with the pharmaceutical composition (KH002). Based on these results, it may be predicted that the treatment with the pharmaceutical composition (KH002) is effective in recovering from inflammation caused by dental caries.

FIG. 2B shows the results of treating a dental caries rat model with a pharmaceutical composition (KH002) at a depth corresponding to human dental caries stage 3, where the results confirmed the number of inflammatory cells infiltrated into the dental pulp to quantify the degree of inflammation in dental caries by comparing ED1, a representative dental pulp inflammatory cell marker, using an immunochemical staining method.

### Example 5: Analysis of the effect on the expression of inflammatory factors in dental caries

To confirm the preventive and therapeutic effects of a pharmaceutical composition (KH002) in stage 4 dental caries in which inflammation occurred after cells are affected by dental caries, human dental pulp cells were treated with lipopolysaccharide (LPS) and the pharmaceutical composition (KH002), and the expression of inflammatory factors was compared.

All experiments using human pulp cells were approved by the Institutional Review Board (IRB; S-D20140007) of the Seoul National University Dental Hospital and conducted in accordance with the guidelines and regulations of the institution. The third molars (wisdom teeth) were extracted from patients who signed the consent forms at the Seoul National University Dental Hospital, and pulp cells were isolated using a previously reported method (Choung et al., 2016). The teeth were cut open, the internal pulp was separated using forceps, and the pulp tissue was finely minced into 60-mm diameter cell culture dishes. The finely minced pulp tissue was then covered with a cover glass and cultured in a cell culture medium to isolate human pulp cells. After inoculating the human pulp cells in a 6-well dish at 1 × 10⁵ cells/well, the negative control group was treated with the culture medium only, while the positive control groups were treated with only 10 µg/mL of LPS and only 10 µg/mL of the pharmaceutical composition (KH002), respectively, and the experimental group was treated with 10 µg/mL of LPS and 10 µg/mL of the pharmaceutical composition (KH002) together to investigate the preventive effect. The experimental group was pretreated with LPS for 24 hours and then treated with the pharmaceutical composition (KH002) to confirm the therapeutic effect.

A reverse transcription polymerase chain reaction (RT-PCR) was performed to compare the expression of inflammatory factors, and the ratio of the expression level of each factor to the expression level of glyceraldehyde 3-phosphate dehydrogenase (GAPDH) was confirmed for quantitative comparison (FIG. 3).

When the human dental pulp cells were treated with LPS only, it was confirmed that the expression of inflammatory factors TNF-α, IL-1α, IL-1β, IL-6, CXCL10, iNOS, and COX2 was significantly increased. On the contrary, when the pharmaceutical composition (KH002) was applied after the LPS pretreatment or when LPS and the pharmaceutical composition (KH002) were treated together, it was confirmed that expression patterns were similar to the negative control group that did not receive any treatment. These results show that the pharmaceutical composition (KH002) has a preventive and therapeutic effect in alleviating inflammation related to dental caries when inflammation occurs after cells are affected by dental caries.

The primer sequences for RT-PCR for each factor are shown below (Table 13).

**[Table 13]**

| Factor | Primer sequence | SEQ ID NO. |
|---|---|---|
| CPNE7 | F: 5'-CGGGACCCATTGACCAAGTC-3' | 97 |
| | R: 5'-CATACACCTCAAACCGTAGCTTC-3' | 98 |
| TNF-α | F: 5'-CCTGGTATGAGCCCATCTATCTG-3'⊚ | 99 |
| | R: 5'-GCAATGATCCCAAAGTAGACCTG-3' | 100 |
| IL-1α | F: 5'-TGTATGTGACTGCCCAAGATGAAG-3' | 101 |
| | R: 5'-AGAGGAGGTTGGTCTCACTACC-3' | 102 |
| IL-1β | F: 5'-CCAGGGACAGGATATGGAGCA-3' | 103 |
| | R: 5'-TTCAACACGCAGGACAGGTACAG-3' | 104 |
| IL-6 | F: 5'-CGAAAGTCAACTCCATCTGCC-3' | 105 |
| | R: 5'-GGCAACTGGCTGGAAGTCTCT-3' | 106 |
| CXCL10 | F: 5'-TGCCATTCTGATTTGCTGCC-3' | 107 |
| | R: 5'-TGCAGGTACAGCGTACAGTT-3' | 108 |
| Inos | F: 5'-CAGCGGGATGACTTTCCAA-3' | 109 |
| | R: 5'-AGGCAAGATTTGGACCTGCA-3' | 110 |
| COX2 | F: 5'-TTCTCCTTGAAAGGACTTATGGGTAA-3' | 111 |
| | R: 5'-AGAACTTGCATTGATGGTGACTGTTT-3' | 112 |

### Example 6: Analysis of the effect on the viability of dentin cells

The effect of a pharmaceutical composition (KH002) on the cell viability rate of human dental pulp cells in an inflammatory environment was confirmed in relation to stage 4 dental caries in which inflammation occurred after the cells were affected by dental caries.

To measure the cell viability, human dental pulp cells were inoculated in a 96-well plate at 5 × 10³ cells/well and treated with 150 µL of a 3-(4,5-dimethyl-2-thiazolyl)-2,5-diphenyl-tetrazolium bromide (MTT, Medifab Co. Ltd.) solution for about two hours on days 1, 3, and 5, and the absorbance of the solution dissolved in DMSO was measured at 570 nm.

All experiments using human pulp cells were approved by the Institutional Review Board (IRB; S-D20140007) of the Seoul National University Dental Hospital and conducted in accordance with the guidelines and regulations of the institution. The third molars (wisdom teeth) were extracted from patients who signed the consent forms at the Seoul National University Dental Hospital, and pulp cells were isolated using a previously reported method (Choung et al., 2016). The teeth were cut open, the internal pulp was separated using forceps, and the pulp tissue was finely minced into 60-mm diameter cell culture dishes. The finely minced pulp tissue was then covered with a cover glass and cultured in a cell culture medium to isolate human pulp cells. After inoculating the human pulp cells in a 6-well dish at 1 × 10⁵ cells/well, the negative control group was treated with the culture medium only, while the positive control groups were treated with only 10 µg/mL of LPS and only 10 µg/mL of the pharmaceutical composition (KH002), respectively, and the experimental group was treated with 10 µg/mL of LPS and 10 µg/mL of the pharmaceutical composition (KH002) together to investigate the preventive effect. The experimental group was pretreated with LPS for 24 hours and then treated with the pharmaceutical composition (KH002) to confirm the therapeutic effect.

FIG. 4 shows a cell experiment simulating a situation where pulp inflammation caused by dental caries corresponding to stage 4 occurs and shows a graph illustrating the effect of treating human dental pulp cells with the pharmaceutical composition (KH002) under an inflammatory environment on the viability of dental pulp cells.

The experimental group treated with the pharmaceutical composition (KH002) at a 10 µg/mL concentration did not show a significant difference from the negative control group. The group treated with 10 µg/mL of LPS showed a significant decrease in the viability of dental pulp cells compared to the negative control group, and the group treated with 10 µg/mL of the pharmaceutical composition (KH002) together with 10 µg/mL of LPS showed a recovery in the viability of dental pulp cells to a similar level as the negative control group.

These results imply that the pharmaceutical composition (KH002) may provide the function of protecting the dental pulp and alleviating inflammation by increasing the viability of human dental pulp cells when inflammation occurs after the cells are affected by dental caries.

### Example 7: Analysis of the effect on the recovery of dental cells

In a wound-healing model of human pulp cells, the effects of an inflammatory environment and pharmaceutical composition (KH002) treatment on wound-healing ability were confirmed.

Human pulp cells were inoculated in a 6-well plate at 1 × 10⁵ cells/well, and the next day, the central portion was scratched with the tip of a 200 µL pipette, and the rate of cell growth was compared 24 hours later.

All experiments using human pulp cells were approved by the Institutional Review Board (IRB; S-D20140007) of the Seoul National University Dental Hospital and conducted in accordance with the guidelines and regulations of the institution. The third molars (wisdom teeth) were extracted from patients who signed the consent forms at the Seoul National University Dental Hospital, and pulp cells were isolated using a previously reported method (Choung et al., 2016). The teeth were cut open, the internal pulp was separated using forceps, and the pulp tissue was finely minced into 60-mm diameter cell culture dishes. The finely minced pulp tissue was then covered with a cover glass and cultured in a cell culture medium to isolate human pulp cells. After inoculating the human pulp cells in a 6-well dish at 1 × 10⁵ cells/well, the negative control group was treated with the culture medium only, while the positive control groups were treated with only 10 µg/mL of LPS and only 10 µg/mL of the pharmaceutical composition (KH002), respectively, and the experimental group was treated with 10 µg/mL of LPS and 10 µg/mL of the pharmaceutical composition (KH002) together to investigate the preventive effect. The experimental group was pretreated with LPS for 24 hours and then treated with the pharmaceutical composition (KH002) to confirm the effect on the recovery of dental pulp cells.

Cell photographs were taken using an optical microscope using a 10x objective lens, and the photographs were analyzed using the ImageJ software program.

FIG. 5 shows a cell experiment simulating a situation where pulp inflammation caused by dental caries corresponding to stage 4 occurs, and illustrates the effect of treating damaged human pulp cells with a pharmaceutical composition according to an embodiment of the present invention under an inflammatory environment on the recovery of the pulp cells.

As a result of quantifying the wound healing ability of the experimental group as a ratio to the control group, the group treated with only the pharmaceutical composition (KH002) did not show a significant difference from the control group, but the wound healing ability of the group treated with LPS significantly decreased to about 50% of the control group. In the group treated with LPS and the pharmaceutical composition (KH002) together, the wound healing ability recovered to about 90%, confirming that the pharmaceutical composition (KH002) restores the wound healing ability of human dental pulp cells even in an inflammatory environment caused by dental caries.

### Example 8: Analysis of the effect on the mineralization ability of pulp cells

The effect of pharmaceutical composition (KH002) treatment on the mineralization ability of human dental pulp cells was confirmed when inflammation occurred after the cells were affected by dental caries.

To this end, human dental pulp cells were inoculated in each 6-well dish at 1 × 10⁵ cells/well and then cultured in a differentiation medium containing ascorbic acid (50 µg/mL) and beta-glycerophosphate (10 mM) for 7, 14, and 21 days from the next day.

All experiments using human pulp cells were approved by the Institutional Review Board (IRB; S-D20140007) of the Seoul National University Dental Hospital and conducted in accordance with the guidelines and regulations of the institution. The third molars (wisdom teeth) were extracted from patients who signed the consent forms at the Seoul National University Dental Hospital, and pulp cells were isolated using a previously reported method (Choung et al., 2016). The teeth were cut open, the internal pulp was separated using forceps, and the pulp tissue was finely minced into 60-mm diameter cell culture dishes. The finely minced pulp tissue was then covered with a covered glass and cultured in a cell culture medium to isolate human pulp cells. After inoculating the human pulp cells in a 6-well dish at 1 × 10⁵ cells/well, the negative control group was treated with the culture medium only, while the positive control groups were treated with only 10 µg/mL of LPS and only 10 µg/mL of the pharmaceutical composition (KH002), respectively, and the experimental group was treated with 10 µg/mL of LPS and 10 µg/mL of the pharmaceutical composition (KH002) together to investigate the preventive effect. The experimental group was pretreated with LPS for 24 hours and then treated with the pharmaceutical composition (KH002) to confirm the effect on the mineralization ability.

To compare the amount of mineralized matrix, the samples were fixed overnight at 4 °C with 4% paraformaldehyde (PFA), then treated with 40 mM alizarin red S (pH 4.2) at 25 °C for 30 minutes. After that, 0.5 mL of sodium dodecyl sulfate dissolved in 0.5 N hydrochloric acid was applied, and the samples were placed on a shaker for 30 minutes, and the absorbance was measured at 405 nm.

FIG. 6 shows a cell experiment simulating a situation when pulp inflammation caused by dental caries corresponding to stage 4 occurs and illustrates the results of examining the effect on the mineralization ability of human pulp cells. Referring to FIG. 6, compared to the control group, the group treated with the pharmaceutical composition (KH002) exhibited an increase in the size of the mineral nodules on day 21 and a significant increase in the amount of mineralized matrix on days 7, 14, and 21. On the contrary, in the group treated with LPS, the size of the mineral nodules on day 21 and the amount of mineralized matrix on days 7, 14, and 21 were significantly decreased compared to the control group. The group treated with LPS and the pharmaceutical composition (KH002) together showed an increase in the size of the mineral nodules on day 21 compared to the group treated with LPS, and the amount of mineralized matrix recovered to a level almost similar to that of the negative control group on days 7 and 14. The experimental group did not recover to the same extent as the control group after 21 days, but it showed a statistically significant increase compared to the group treated with only LPS. These results confirmed that the pharmaceutical composition (KH002) may restore and improve the dentin secretion ability of odontoblasts in human dental pulp cells in an inflammatory environment caused by dental caries, thereby providing an effect of restoring the thickness of damaged dentin.

The pharmaceutical composition according to an embodiment of the present invention can be used in the prevention and treatment of various dental caries diseases, such as preventing root caries in the elderly to whom general dental caries treatment methods are difficult to apply, and maintaining the deciduous teeth of children with dental caries until they fall out.

In addition, the pharmaceutical composition according to an embodiment of the present invention can protect odontoblasts even in cases where inflammation has occurred in a severe dental caries stage that cannot be restored to a natural tooth state with general dental caries treatment, and can provide the effect of treating dental caries through the protection of dental pulp cells by allowing the autophagy function of newly formed odontoblasts to also be provided.

The present specification and drawings have disclosed preferred embodiments of the present invention, and although specific terms have been used, they are used only in a general sense to easily explain the technical contents of the present invention and to help understand the invention and are not intended to limit the scope of the present invention. It will be obvious to those skilled in the art that other modifications based on the technical spirit of the present invention can be implemented in addition to the embodiments disclosed herein.

## Claims

1. A pharmaceutical composition for preventing or treating dental caries, comprising a peptide consisting of an amino acid sequence of General Formula 1 below as an active ingredient:
K-Y-R1-R2-R3-R4-R5-R6-R7-R8 (General Formula 1)
in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).

2. The pharmaceutical composition of claim 1, wherein the peptide consists of any one of the amino acid sequences of SEQ ID NOs: 1 to 96.

3. The pharmaceutical composition of claim 1, wherein the pharmaceutical composition includes a polypeptide in which the peptide is repeatedly linked.

4. The pharmaceutical composition of claim 1, wherein the pharmaceutical composition includes a complex in which a drug having an antibiotic effect against oral bacteria is combined with the peptide.

5. The pharmaceutical composition of claim 1, wherein the pharmaceutical composition further includes a pharmaceutically acceptable carrier, excipient, or diluent.

6. A quasi-drug composition for preventing or ameliorating dental caries, comprising the peptide of claim 1 or claim 2.

7. A healthy functional food for preventing or ameliorating dental caries, comprising the peptide of claim 1 or claim 2.

8. A method for preventing or treating dental caries, comprising administering to an individual other than a human a pharmaceutical composition for preventing or treating dental caries, including a peptide consisting of an amino acid sequence of General Formula 1 below as an active ingredient:
K-Y-R1-R2-R3-R4-R5-R6-R7-R8 (General Formula 1)
in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).
